# EUROPEAN PATENT SPECIFICATION

(11) **EP 2 936 435 B1**
(45) Date of publication and mention of the grant of the patent: **05.06.2019**
(21) Application number: 13822020.7
(22) Date of filing: 18.12.2013
(51) Int. Cl.: G01N 33/00, G06T 7/00

(54) **NON-DESTRUCTIVE IMAGING OF CROP PLANTS**
ZERSTÖRUNGSFREIE ABBILDUNG VON ZUCHTPFLANZEN
IMAGERIE NON DESTRUCTRICE DE PLANTES EN CULTURE

(30) Priority: 20.12.2012 US 201261740208 P
(43) Date of publication of application: 28.10.2015
(73) Proprietor: Pioneer Hi-Bred International, Inc., Des Moines, IA 50309 (US)
(72) Inventor: COPE, Jason, Ankeny, Iowa 50023 (US); LI, Guofu, Johnston, Iowa 50131 (US); MORIARTY, Timothy, Michael, Urbandale, Iowa 50322 (US)
(74) Representative: Dehns
(86) International application number: PCT/US2013/076235
(87) International publication number: WO 2014/100237

(56) References cited:
- US-A1- 2011 135 161
- US-A1- 2011 167 721
- ROSELL J R ET AL: "A review of methods and applications of the geometric characterization of tree crops in agricultural activities", COMPUTERS AND ELECTRONICS IN AGRICULTURE, ELSEVIER, AMSTERDAM, NL, vol. 81, 16 September 2011 (2011-09-16), pages 124-141, XP028442024, ISSN: 0168-1699, DOI: 10.1016/J.COMPAG.2011.09.007 [retrieved on 2011-09-22]
- HEERAMAN D A ET AL: "Three dimensional imaging of plant roots in situ with X-ray computed tomography", PLANT AND SOIL, KLUWER ACADEMIC PUBLISHERS, DORDRECHT, NL, vol. 189, no. 2, 1 January 1997 (1997-01-01), pages 167-179, XP002170638,

## Description

### FIELD OF THE DISCLOSURE

The disclosure relates to the field of plant biotechnology and concerns yield assessment of crop plants.

### BACKGROUND OF THE DISCLOSURE

Evaluation of yield and/or of stress tolerance in crop plants such as maize and soybean usually occurs by way of field testing (e.g. in a yield trial). However, field testing requires significant time, manpower, acreage, and monetary resources, which hinders the number of plants that can be evaluated in any given period of time. The problem remains as to how to rapidly assess plants for yield or yield-related traits using fewer resources and preferably, in a non-destructive manner. X ray imaging is a suitable method to analyze traits in crop plants. US2011167721 discloses a plant evaluation system in which the plant is imaged (possibly using X-rays) and the resulting images are evaluated to determine phenotype characteristics such as prospective yield or stress tolerance.

### SUMMARY OF THE DISCLOSURE

The methods presented herein use X ray imaging and processing technologies to non-destructively assess yield and/or stress tolerance in crop plants. In corn, for example, ear length, number of seeds per ear, anthesis, and silking can be non-destructively imaged and analyzed using high energy particles or sources such as X rays.

A method for non-destructively evaluating a crop plant for yield and/or for stress tolerance is provided. In the method, multiple two-dimensional (2-D) X ray images of the crop plant or a part thereof are acquired using an X ray imaging system; the images or a select sampling of the images are processed into a single composition (referred to as a voxel) using computed tomography to generate a three-dimensional (3-D) image; one or more physical properties of the plant or part thereof are determined using the three-dimensional image; and the crop plants are evaluated for yield and/or for stress tolerance based on the one or more physical properties. Alternatively, the one or more physical properties of the plant or part thereof may be determined using one or more of the 2-D images (in the absence of processing) and the crop plants may be evaluated based on such.

The crop plant may be maize, soybean, sorghum, canola, wheat, rice, or barley, and the crop plant may or may not contain a transgene of interest. The images of the crop plant or part thereof may be acquired at any point in a crop plant's life cycle including but not limited to the crop plant's reproductive stage(s).

The crop plant may be maize and a physical property of the maize plant may be ear area, ear length, ear width, ear diameter, ear perimeter, number of seeds per ear, or seed size. The crop plant may be soybean and a physical property of the soybean plant may be pods per plant, number of seeds per pod, or seed size.

The method may further comprise comparing said crop plant to another crop plant of the same species based on the one or more physical properties for the purpose of plant selection and advancement as part of a plant development program.

A method for non-destructive, high-throughput analysis of the effect of a transgene of interest on yield and/or on stress tolerance in a crop plant is also provided herein. In the method, a population of transgenic crop plants is provided; multiple two-dimensional X ray images of the transgenic crop plants or parts thereof are acquired using an X ray imaging system; the acquired images are processed using computed tomography to generate three-dimensional images; a mean or median value is calculated and a coefficient of variation is obtained for the population of transgenic crop plants with respect to the one or more physical properties based on the three-dimensional images; and a statistical test is performed to determine if there is a significant difference between a single transgenic crop plant and the population of transgenic crop plants for one or more physical properties.

The transgenic crop plant may be maize, soybean, sorghum, canola, wheat, rice, or barley. The images of the crop plant may be acquired at any point in a crop plant's life cycle including but not limited to the crop plant's reproductive stage(s).

The transgenic crop plant may be maize and a physical property of the maize plant may be ear area, ear length, ear width, ear diameter, ear perimeter, number of seeds per ear, or seed size. The transgenic crop plant may be soybean and a physical property of the soybean plant may be pods per plant, number of seeds per pod, or seed size.

### DETAILED DESCRIPTION

The disclosure of each reference set forth herein is hereby incorporated by reference in its entirety.

As used herein and in the appended claims, the singular forms "a", "an", and "the" include plural reference unless the context clearly dictates otherwise. Thus, for example, reference to "a plant" includes a plurality of such plants, reference to "a cell" includes one or more cells and equivalents thereof known to those skilled in the art, and so forth.

### Overview

In many crop plants, typical methods for the assessment of plant properties associated with yield and/or with stress tolerance require significant resources (i.e. labor, acreage, etc.) and destruction of the plant. In maize, for example, the ear is concealed by the husk, so in order to evaluate properties such as but not limited to ear area, ear length, ear width, ear diameter, ear perimeter, number of seeds per ear, or seed size, the ear is removed from the husk and/or the seeds are removed from the cob. In soybeans, the complex architecture, which is characterized by multiple seed pods that are distributed across and within a soybean plant's foliage, requires that drying, threshing, cleaning, and mechanical seed counting be included in the process when assessing properties such as seed number, seed size, number of seeds per pod, etc. Any crop for which the seeds are encased in a protective structure such as a husk, pod, etc. faces the same obstacles in the assessment of yield.

X rays can penetrate plant tissues and therefore allow visualization of concealed and/or internal plant parts. The use of X ray imaging and analysis techniques (such as X ray computed tomography) as presented herein provide a precise and rapid analytical method to assess yield and/or stress tolerance in a non-destructive manner at any point in a crop plant's life cycle but especially in the crop plant's reproductive stage(s).

### X ray Imaging

X ray computed tomography (CT) is an imaging procedure that utilizes computer-processed X rays to produce tomographic images or 'slices' of an object. X ray slice data is generated using an X ray imaging system that consists of an X ray source or generator (X ray tube) and an image detection system which can be either a film (analog technology) or a digital capture system. The X ray imaging system may include a fixed scanner or a portable scanner.

Individual cross sections can be acquired (also referred to as axial slices) as well as continuously changing cross sections (also referred to as a helical scan). The latter can be performed using a helical or spiral CT machine, otherwise referred to as a spinning tube. In a spinning tube, an entire X ray tube is spun around the central axis of the area being scanned.

Computer systems integrate data of the individual slices to generate three dimensional volumetric information, or voxel. Once the scan data is acquired, the data must be processed using a form of tomographic reconstruction, which produces a series of cross-sectional images. Tomographic reconstruction can be performed using any method known to one of ordinary skill in the art including but not limited to: filtered back projection, linear algebra, iterative physical model-based maximum likelihood expectation maximization techniques, multiplanar reconstruction, and 3-D rendering techniques such as surface rendering, volume rendering, or image segmentation.

With multiplanar reconstruction, a volume is built by stacking the axial slices, and with the aid of software, slices can be made through the volume in a different plane.

Surface rendering involves a threshold value of radiodensity set by the operator, upon which a three-dimensional model can be constructed using edge detection image processing algorithms.

In volume rendering, transparency and colors are used to allow a better representation of the volume to be shown in a single image.

Image segmentation is the process of partitioning a digital image into multiple segments. Image segmentation modifies the representation of an image so that it is more meaningful and easier to analyze. With image segmentation, a label is assigned to every pixel in an image. "Binary segmentation", for example, involves setting a pixel on or off depending on how it compares to a pre-selected threshold level.

### Acquisition of Plant Images

The crop plant may be maize, soybean, sorghum, canola, wheat, rice, or barley. The part can be an ear, a pod, a branch, a seed head, a spikelet or spike, a panicle, or any seed bearing structure.

Plants that are to be imaged may be grown in a "controlled environment setting", such as a greenhouse or growth chamber, where water and nutrient availability is controlled as are other factors including but not limited to: temperature, exposure to extreme weather elements, and pests, or in a screenhouse or field environment in which there is little to no control over environmental effects.

Plants may be grown using any of a number of experimental designs that will reduce or eliminate sources of experimental error. Some examples of designs include but are not limited to: one-factor designs, nested designs, factorial designs, randomized block designs, split plot designs, repeated measure designs, and unreplicated designs. One of ordinary skill in the art would be familiar with these and other experimental designs.

"Non-destructive" generally refers to a plant or plant part that is not harvested or is not permanently removed from its growth media. The growth media may include but is not limited to field soil, potting soil, water (as in hydroponics), sand, gravel, and any other media used for growing crop plants that is known to one of ordinary skill in the art. "Non-destructive" can also pertain to a part of the crop plant, wherein the part that is being imaged is not physically separated from the crop plant.

The X ray imaging system may be transported to the site of the plant or plant part, or the plant or plant part may be transported to the X ray imaging system.

The images of the crop plant may be acquired at any point in a crop plant's life cycle including but not limited to a crop plant's reproductive stage(s). The reproductive stages of plants are well known to one of ordinary skill in the art.

Plants may contain a transgene of interest and are otherwise referred to herein as "transgenic plants". The term "transgenic plant" refers to a plant which comprises within its genome one or more heterologous polynucleotides. For example, the heterologous polynucleotide is stably integrated within the genome such that the polynucleotide is passed on to successive generations. The heterologous polynucleotide may be integrated into the genome alone or as part of a recombinant DNA construct. Each heterologous polynucleotide may confer a different trait to the transgenic plant.

Each plant may be identified and tracked through the entire process, and the data gathered from each plant may be automatically associated with that plant. For example, each plant may have a machine-readable label (such as a Universal Product Code (UPC) bar code) which may include information about the plant identity and location in the field or greenhouse.

One or more plants or a plant population in a controlled environment setting such as a greenhouse may be transported in a high-throughput fashion automatically to an imaging location where X ray images are taken at a whole plant level. For example, the whole maize plant or soybean plant may be non-destructively imaged, but the analysis of any 2-D or 3-D X ray images may be limited to ears or pods.

### Associating Physical Properties with Yield and/or with Stress Tolerance

A "physical property" of a crop plant or part thereof generally refers to a measurable parameter. One or more physical properties may directly contribute to grain yield. Physical properties of maize ears that correlate with yield, for example, include but are not limited to ear area, ear length, ear width, ear diameter, ear perimeter, number of seeds per ear, and seed size, whereas in legumes such as soybeans, the number of pods per plant, the number of seeds per pod, and seed size correlate with yield. Physical properties of other structures such as leaf or stem structures may also be evaluated; these may include but are not limited to leaf angle, leaf area, internode distance, etc.

Crop plants may also be grown in or subjected to a stress environment. Thus, assessing a physical property of a crop plant under such conditions using the methods of the invention allows one to make determinations regarding the stress tolerance of crop plants (i.e. how the crop plants perform with respect to yield when exposed to stress).

Stress tolerance may be tolerance to an abiotic stress or a biotic stress. An "abiotic stress" may be at least one condition selected from the group consisting of: drought, water deprivation, flood, high light intensity, high temperature, low temperature, salinity, etiolation, defoliation, heavy metal toxicity, anaerobiosis, nutrient deficiency, nutrient excess, UV irradiation, atmospheric pollution (e.g., ozone) and exposure to chemicals (e.g., paraquat) that induce production of reactive oxygen species (ROS). A "biotic stress" refers to a stress that occurs as a result of damage done to plants by other living organisms, such as bacteria, viruses, fungi, parasites, beneficial and harmful insects, weeds, and cultivated or native plants.

For example, plants may be grown under water limiting conditions. "Water limiting conditions" refers to a plant growth environment where the amount of water is not sufficient to sustain optimal plant growth and development. One skilled in the art would recognize conditions where water is sufficient to sustain optimal plant growth and development. The terms "drought" and "water limiting conditions" are used interchangeably herein.

When a genotype yields better than another under water-limiting conditions, the plant is generally referred to as being "drought tolerant." "Drought tolerance" is a trait of a plant to survive under drought conditions over prolonged periods of time without exhibiting substantial physiological or physical deterioration. "Drought" refers to a decrease in water availability to a plant that, especially when prolonged, may cause damage to the plant or prevent its successful growth (e.g., limiting plant growth or seed yield).

A "drought tolerant crop plant" is a crop plant that exhibits drought tolerance. A drought tolerant crop plant may be a plant that exhibits an increase in at least one physical property of the plant, as compared to a control plant under water limiting conditions.

One of ordinary skill in the art is familiar with protocols for simulating drought conditions and for evaluating drought tolerance of plants that have been subjected to simulated or naturally-occurring drought conditions. For example, one may simulate drought conditions by giving plants less water than normally required or no water over a period of time. A drought stress experiment may involve a chronic stress (i.e., slow dry down) and/or may involve two acute stresses (i.e., abrupt removal of water) separated by a day or two of recovery. Chronic stress may last 8 - 10 days. Acute stress may last 3 - 5 days.

Plants may be grown under nitrogen limiting conditions. "Nitrogen limiting conditions" refers to a plant growth environment where the amount of total available nitrogen (e.g., from nitrates, ammonia, or other known sources of nitrogen) is not sufficient to sustain optimal plant growth and development. One skilled in the art would recognize conditions where total available nitrogen is sufficient to sustain optimal plant growth and development. One skilled in the art would also recognize what constitutes sufficient amounts of total available nitrogen, and what constitutes soils, media and fertilizer inputs for providing nitrogen to plants. Nitrogen limiting conditions will vary depending upon a number of factors, including but not limited to, the particular plant and environmental conditions.

When a genotype yields better than another under nitrogen limiting conditions, the plant is generally referred to as being "nitrogen stress tolerant." "Nitrogen stress tolerance" is a trait of a plant and refers to the ability of the plant to survive under nitrogen limiting conditions.

A "nitrogen stress tolerant plant" is a plant that exhibits nitrogen stress tolerance. A nitrogen stress tolerant plant may be a plant that exhibits an increase in at least one physical property of a maize plant, for example, relative to a control maize plant under nitrogen limiting conditions.

One of ordinary skill in the art is familiar with protocols for simulating nitrogen stress conditions and for evaluating nitrogen stress tolerance of plants that have been subjected to simulated or naturally-occurring nitrogen limiting conditions.

Plants may also be evaluated for tolerance to biotic stresses such as a bacteria or virus . For example, insect or disease damage to reproductive structures such as corn ears or soybean pods or to other plant structures such as leaves or roots can be evaluated using the methods disclosed herein.

### Data Evaluation

The data may be paired with other data so that relationships between the pairs of data may be determined by regression or other statistical techniques used to relate sets of variables. It is to be understood that the type of relationship present between pairs of data may vary and as such different mathematical or statistical tools may be applied. It is to be understood also, that instead of relating two sets of data (pairing), multiple sets of data may be related.

The data extracted from the images may be used to quantify within-plot variability. A "plot" is simply an area where multiple plants of similar genetic background are grown. Within-plot variability describes variations between plants within the plot. Examples of types of within-plot variability measurements include, without limitation, standard error, standard deviation, relative standard deviation, skew, kurtosis, variance, coefficient of variation, and interquartile range.

In one aspect, mean or median values of a physical property (or physical properties) are calculated, as well as a coefficient of variation, for a population of transgenic plants, and a statistical test is performed to determine if there is a significant difference between the mean or median of a single member of the population of transgenic plants as compared to the mean or median value for the population of transgenic plants with respect to the physical property (or properties). The difference may be considered attributable to the transgene of interest. Transgene effect can be measured early in the transgenic variety development process, e.g. as early as the T0 and T1 generations, thereby eliminating the need to generate seed necessary for multi-location replicated field trials. Moreover, the effect can be evaluated under a variety of environmental conditions (e.g. optimal or stress induced environments). Evaluation of transgene effects can be accomplished on a large scale - thousands to tens of thousands of genes per year, at a dramatically lower cost (because of reduced manpower and field resources), and far more quickly than traditional transgene function testing methods (such as, e.g. in yield trials).

### Repeated measurements

Since it is not necessary for the plant structure being imaged to be removed or modified, non-destructive X ray imaging allows for a repeated measuring of yield and/or of stress tolerance at different stages of development. With respect to maize ears, for example, multiple images of the same ear(s) could be obtained over the course of ear growth and development, thereby allowing ear growth rate and/or seed growth rate to be assessed. Similar analyses could be performed with soybean, in which pod growth rate or seed growth rate could be assessed.

Moreover, the ability to evaluate crop plants and/or parts thereof non-destructively at multiple points in a crop plant's life cycle enables one of ordinary skill in the art to assess the overall state of the plant with respect to the effect of stresses incurred on the plant, biomass, shape, and general health characteristics. The methods thus enhance crop plant development processes by allowing for the healthiest and most desirable plants to be identified and selected for advancement.

### EXAMPLES

The following examples are offered to illustrate, but not to limit, the claimed disclosure. It is understood that the examples and embodiments described herein are for illustrative purposes only, and persons skilled in the art will recognize various reagents or parameters that can be altered without departing from the scope of the appended claims.

### EXAMPLE 1

### NON-DESTRUCTIVE COLLECTION OF MAIZE EAR PROPERTIES USING X RAY IMAGING

Measurement of the physical properties of a maize plant or part thereof can be carried out in a destructive or a non-destructive manner by use of an X ray imaging system. For example, two-dimensional scans of maize ears can be obtained using an X ray imaging system. Two dimensional images can be obtained from X ray scanning, and the images can be processed using computed tomography. Physical properties of the maize plant and/or ear such as but not limited to ear area, ear length, ear width, ear diameter, ear perimeter, number of seeds per ear, and seed size can be evaluated using the three-dimensional projection.

### EXAMPLE 2

### NON-DESTRUCTIVE COLLECTION OF SOYBEAN PHYSICAL PROPERTIES USING X RAY IMAGING

Measurement of the physical properties of a soybean plant or part thereof can be carried out in a destructive or a non-destructive manner by use of an X ray imaging system. Two dimensional images can be obtained from X ray scanning, and the images can be processed using computed tomography to get a projection of a soybean plant or a part thereof. Physical properties of the soybean plant and/or such as but not limited to the number of pods per plant, the number of seeds per pod, or seed size can be evaluated using the three-dimensional projection.

## Claims

1. A method of non-destructively evaluating a maize plant for yield and/or for stress tolerance comprising:
a. acquiring multiple two-dimensional X ray images of an ear of the maize plant using an X ray imaging system;
b. processing the images or a select sampling of the images using computed tomography to generate a three-dimensional image;
c. determining at least one physical property of the ear of the maize plant from the three-dimensional image; and
d. evaluating the maize plant for yield and/or for stress tolerance based on the at least one physical property.

2. The method of claim 1, wherein the maize plant comprises a transgene of interest.

3. The method of claim 1, wherein the stress tolerance is one of nitrogen stress tolerance, drought tolerance, tolerance to insect pests, or tolerance to disease.

4. The method of claim 1, wherein the at least one physical property is one of ear area, ear length, ear width, ear diameter, ear perimeter, number of seeds per ear, or seed size.

5. The method of claim 1, wherein said maize plant was grown under water-limiting or nitrogen-limiting conditions.

6. The method of claim 1, further comprising comparing said maize plant to another maize plant based on the at least one physical property.

## Patentansprüche

1. Verfahren zum nichtdestruktiven Evaluieren einer Maispflanze auf Ertrag und/oder Stresstoleranz, umfassend:
a) Aufnehmen mehrerer zweidimensionaler Röntgenbilder eines Kolbens der Maispflanze unter Verwendung eines Röntgenabbildungssystems;
b) Verarbeiten der Bilder oder einer ausgewählten Probenahme der Bilder, unter Verwendung von Computertomografie, um ein dreidimensionales Bild zu erzeugen;
c) Ermitteln mindestens einer physikalischen Eigenschaft des Kolbens der Maispflanze aus dem dreidimensionalen Bild; und
d) Evaluieren der Maispflanze auf Ertrag und/oder Stresstoleranz, basierend auf der mindestens einen physikalischen Eigenschaft.

2. Verfahren nach Anspruch 1, wobei die Maispflanze ein Transgen von Interesse umfasst.

3. Verfahren nach Anspruch 1, wobei die Stresstoleranz eines von Stickstoffstresstoleranz, Dürretoleranz, Toleranz gegenüber Schadinsekten oder Toleranz gegenüber Erkrankung ist.

4. Verfahren nach Anspruch 1, wobei die mindestens eine physikalische Eigenschaft eines von Kolbenfläche, Kolbenlänge, Kolbenbreite, Kolbendurchmesser, Kolbenumfang, Zahl an Samen pro Kolben oder Samengröße ist.

5. Verfahren nach Anspruch 1, wobei die Maispflanze unter wasserbegrenzten oder stickstoffbegrenzten Bedingungen gezogen wurde.

6. Verfahren nach Anspruch 1, weiter umfassend Vergleichen der Maispflanze mit einer anderen Maispflanze, basierend auf der mindestens einen physikalischen Eigenschaft.

## Revendications

1. Procédé d'évaluation non destructive d'un plant de maïs pour le rendement et/ou pour la tolérance au stress comprenant :
a. l'acquisition de multiples images radiographiques bidimensionnelles d'un épi du plant de maïs en utilisant un système d'imagerie radiographique ;
b. le traitement des images ou d'un échantillon sélectionné des images en utilisant la tomodensitométrie pour générer une image tridimensionnelle ;
c. la détermination d'au moins une propriété physique de l'épi du plant de maïs à partir de l'image tridimensionnelle ; et
d. l'évaluation du plant de maïs pour le rendement et/ou pour la tolérance au stress sur la base de la au moins une propriété physique.

2. Procédé selon la revendication 1, dans lequel le plant de maïs comprend un transgène d'intérêt.

3. Procédé selon la revendication 1, dans lequel la résistance au tolérance est une parmi la tolérance au stress azoté, la tolérance à la sécheresse, la tolérance aux insectes nuisibles, ou la tolérance aux maladies.

4. Procédé selon la revendication 1, dans lequel la au moins une propriété physique est une parmi la surface de l'épi, la longueur de l'épi, la largeur de l'épi, le diamètre de l'épi, le périmètre de l'épi, le nombre de graines par épi, ou la taille de graine.

5. Procédé selon la revendication 1, dans lequel ledit plant de maïs a été cultivé dans des conditions limitant l'eau ou limitant l'azote.

6. Procédé selon la revendication 1, comprenant en outre la comparaison dudit plant de maïs avec un autre plant de maïs sur la base de la au moins une propriété physique.
